# EUROPEAN PATENT SPECIFICATION

(11) **EP 2 948 075 B1**
(45) Date of publication and mention of the grant of the patent: **15.07.2020**
(21) Application number: 14743260.3
(22) Date of filing: 23.01.2014
(51) Int. Cl.: A61B 17/3205, A61B 18/14, A61B 90/00, A61B 18/00

(54) **SURGICAL SNARE DEVICE**
CHIRURGISCHE SCHLINGENVORRICHTUNG
DISPOSITIF D'ANSE CHIRURGICAL

(30) Priority: 23.01.2013 US 201361755971 P
(43) Date of publication of application: 02.12.2015
(73) Proprietor: Boston Scientific Scimed, Inc., Maple Grove, MN 55311 (US)
(72) Inventor: JACOBS, Charlie, Loganville, GA 30052 (US); COX, Lanita, Suwanee, GA 30024 (US); LYNCH, Brian, Huntington, NY 11743 (US)
(74) Representative: Vossius & Partner Patentanwälte Rechtsanwälte mbB
(86) International application number: PCT/US2014/012826
(87) International publication number: WO 2014/116877

(56) References cited:
- US-A- 5 984 920
- US-A1- 2005 228 403
- US-A1- 2007 255 289
- US-A1- 2008 033 238
- US-A1- 2009 112 226
- US-A1- 2012 289 945
- US-B1- 6 352 539

## Description

### FIELD

The present specification relates generally to surgical instruments. More particularly, the present specification relates to a surgical snare device used to remove polyps wherein the snare is capable of being deployed with a plurality of precise loop diameters.

### BACKGROUND

Surgical snares are used to remove small masses of tissue that project outward or upward from the normal surface level of hollow organs or body cavities. These masses of tissue are referred to as polyps and the procedure to remove them using a surgical snare is termed a polypectomy. Polypectomy procedures are often performed in the colon. A polypectomy snare device typically includes a long, thin sheath with a handle at its proximal end and a flexible wire within the sheath that terminates in a snare loop. While within the sheath, the wire loop is stored in a closed, compressed configuration. The wire loop can be advanced through an opening at the distal end of the sheath by manipulating an actuator on the handle. As the wire loop is advanced beyond the distal end of the sheath, it expands into an open configuration.

During a polypectomy procedure, the sheath of the surgical snare device (with the snare loop retracted within) is inserted into a working channel of an endoscope. The surgeon advances the sheath until its distal end passes beyond the distal end of the endoscope. The surgeon then positions the distal end of the sheath proximate a target polyp. An assistant then manipulates the handle to advance the wire loop out of the sheath. With the loop now in the open, expanded configuration, the surgeon passes the loop over and around the polyp such that the wire loop encircles the base or stalk of the polyp. The assistant then manipulates the handle in an opposite direction to draw the wire loop back into the sheath. At the same time, an electrical current is supplied to the wire to sever and cauterize the polyp. The severed polyp can then be removed using the snare device or with a separate surgical instrument.

Current snare devices include a wire loop having a specific size and shape. For example, the wire loop can have an oval, circular, or hexagonal shape. When fully extended, a particular wire loop can have an internal diameter, or maximum loop width, of, for example, 13, 27, or 30 mm. United States Patent Number 8,162,938, assigned to Boston Scientific Scimed, Inc., describes "A medical device, comprising: a sheath having a proximal end, a distal end, and a lumen defined therein; a shaft slidably disposed in the lumen, the shaft having a distal end; an end effector coupled to the distal end of the shaft with a crimp band; a coupling member adhered to the shaft, disposed over the crimp band, and positioned adjacent the end effector, the coupling member having a distal region that extends distally of the distal end of the sheath when the end effector is deployed, and the coupling member having a proximal region that extends proximally of the distal end of the sheath when the end effector is deployed; and wherein the coupling member comprises a shrink tube."

Patent document US2007/0255289 A1 discloses a snare device with rotatable operative tip and markings on the device handle.

Patent document US6352539 B1 discloses a surgical snare instrument with a rotatable snare loop.

Patent document US2005/0228403 A1 discloses a tissue cutting device with adjustable loop shape.

While current snare devices are effective in removing polyps, they are not without their drawbacks. The exact size of the internal diameter of the wire loop can only be known when the wire loop is fully extended. For example, if a surgeon is using snare device having a 30 mm wire loop, the surgeon can only be certain of the loop's internal diameter when it is fully extended at 30 mm. Less than maximum extension of the 30 mm loop will result in a loop with an internal diameter less than 30 mm, but the exact diameter will not be known, will not be related to the amount of actuation of the handle applied by the physician, and will not be easy to repeatably match that diameter. Therefore, if a surgeon wishes to use a snare loop with a different diameter, he will have to switch out his current snare device for a separate snare device designed with a wire loop having that diameter. This requires a surgeon to have a stock of multiple snare devices with different sizes on hand during a procedure. In addition, current snares tend to become deformed after multiple extensions out of and retractions into the sheath. Multiple passages into and out of the sheath are necessary during multiple polypectomies in a single procedure and have a "banana effect" on the loop, wherein the loop becomes narrower and elongated, losing its oval or circular shape.

Therefore, what is needed is a surgical snare device having a wire loop that can be repeatedly and precisely extended to multiple different internal diameters wherein the device includes a mechanism to indicate the exact diameter to the surgeon. What is also needed is a surgical snare device having a resilient wire loop that can be repeatedly withdrawn into and extended from the sheath without losing its open configuration shape.

### SUMMARY

The present specification discloses a surgical snare device for use with an endoscope according to independent claim 1.

The aforementioned and other embodiments of the present invention shall be described in greater depth in the drawings and detailed description provided below.

### BRIEF DESCRIPTION OF THE DRAWINGS

These and other features and advantages of the present invention will be further appreciated, as they become better understood by reference to the detailed description when considered in connection with the accompanying drawings:
FIG. 1 is an illustration of one embodiment of the snare device of the present specification with the snare loop withdrawn into the sheath;
FIG. 2 is a side view illustration of the embodiment of the snare device of FIG. 1 with the snare loop withdrawn into the sheath, depicting measurement markings printed on the side of the first handle assembly;
FIG. 3 is a side view illustration of the embodiment of the snare device of FIG. 1 with the snare loop withdrawn into the sheath, depicting the collar positioned just distal to the '10' marking on the first handle assembly;
FIG. 4 is a side view illustration of the embodiment of the snare device of FIG. 1 with the snare loop deployed, depicting the second handle assembly advanced to the '10' marking on the first handle assembly;
FIG. 5 is a side view illustration of the embodiment of the snare device of FIG. 1 with the snare loop withdrawn into the sheath, depicting the collar positioned just distal to the '20' marking on the first handle assembly;
FIG. 6 is a side view illustration of the embodiment of the snare device of FIG. 1 with the snare loop deployed, depicting the second handle assembly advanced to the '20' marking on the first handle assembly;
FIG. 7 is a side view illustration of the embodiment of the snare device of FIG. 1 with the snare loop withdrawn into the sheath, depicting the collar positioned just distal to the '30' marking on the first handle assembly;
FIG. 8 is a side view illustration of the embodiment of the snare device of FIG. 1 with the snare loop deployed, depicting the second handle assembly advanced to the '30' marking on the first handle assembly; and,
FIG. 9 is a flowchart illustrating one embodiment of the steps included during a multiple polypectomy procedure using the surgical snare device of the present specification.

### DETAILED DESCRIPTION

The present specification discloses a surgical snare device having a snare loop that is deployable across a range of internal diameters in a precise and controlled manner. The present invention is directed toward multiple embodiments. The following disclosure is provided in order to enable a person having ordinary skill in the art to practice the invention. Language used in this specification should not be interpreted as a general disavowal of any one specific embodiment or used to limit the claims beyond the meaning of the terms used therein. The general principles defined herein may be applied to other embodiments and applications without departing from the scope of the invention. Also, the terminology and phraseology used is for the purpose of describing exemplary embodiments and should not be considered limiting. Thus, the present invention is to be accorded the widest scope encompassing numerous alternatives and modifications consistent with the principles and features disclosed. For purpose of clarity, details relating to technical material that is known in the technical fields related to the invention have not been described in detail so as not to unnecessarily obscure the present invention.

The device includes a handle and an elongated tubular sheath attached to the distal end of the handle. The handle comprises a first handle assembly, a second handle assembly, a cautery tip housing, and a collar. The first handle assembly includes a cylindrical body with a proximal end and a distal end, a finger ring at the proximal end, and a channel extending from a point proximate its proximal end to a point proximate its distal end. The cautery tip housing is attached to and rotatable coaxially about, the distal end of the first handle assembly. The second handle assembly includes a center body portion having a proximal end and a distal end with a finger ring positioned on both sides. The second handles assembly and collar are coupled to, and slidably movable along via the channel of, the first handle assembly. The collar includes a proximal end and a distal end and is positioned distal to the second handle assembly. A rigid driveshaft having a proximal end and a distal end is positioned within the channel and has its proximal end attached to the distal end of the center body of the second handle assembly. The shaft extends distally through the channel, into the cautery tip housing, wherein it is in electrical contact with the cautery tip, and is attached at its distal end to the proximal end of a drive wire. An elongate tubular sheath is attached to the distal end of the first handle assembly and extends distally from the handle. A flexible drive wire extends distally within the tubular sheath and has a snare loop attached at its distal end. The proximal end of the flexible drive wire is permanently fixed to the rigid driveshaft. The snare loop rests within the sheath in a collapsed configuration and is deployable, by sliding the second handle assembly distally along the first handle assembly, to an expanded configuration out of the distal end of the sheath.

The first handle assembly includes a plurality of numerical markings along its length. The markings correspond precisely to the internal diameter or width in millimeters of the deployed snare loop when the distal end of the center body of the second handle assembly aligns with said marking. Therefore, when the distal end of the center body of the second handle assembly is aligned with a '10', the deployed snare loop will have an internal diameter of 10 mm. When the distal end of the center body of the second handle assembly is aligned with a '20', the deployed snare loop will have an internal diameter of 20 mm, and so forth. The collar is included to provide a depth stop for the second handle assembly at the desired marking. Therefore, when a user desires to have a snare loop with an internal diameter of 10 mm, the collar is slid along the first handle assembly such that its proximal end aligns with the '10' marking so that the second handle assembly, when slid distally, will come to rest with its distal end against the proximal end of the collar.

In one embodiment, the drive wire and snare loop are comprised of multiple braided wires. The snare loop has a teardrop shape with a v-shaped notch in its distal end. The wires of the device are annealed at a precise temperature for a specific amount of time to produce a stable loop shape. The snare loop shape and manufacturing process ensure a flat loop deployment and retraction, thereby reducing the need for repositioning the endoscope during deployment and optimizing precise resection during retraction. The snare loop design also enhances tactile feel during resection, reducing inadvertent premature shearing and enhancing user comfort and efficiency. The teardrop configuration serves to maximize the snare loop internal diameter while minimizing the snare loop length, thereby providing greater maneuverability in confined anatomy. The teardrop configuration also reduces the handle throw, enhancing user comfort.

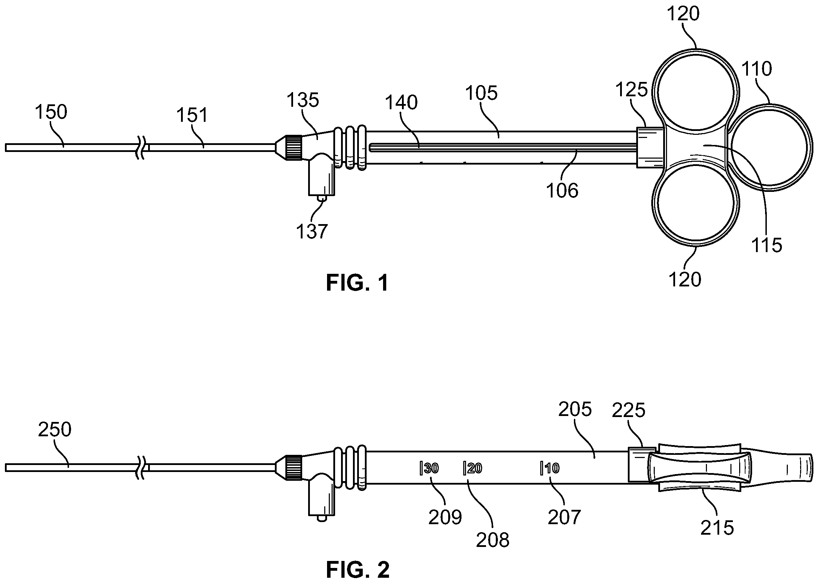

FIG. 1 is an illustration of one embodiment of the snare device of the present specification with the snare loop withdrawn into the sheath 150. In the pictured embodiment, the snare device includes a first handle assembly 105 and a second handle assembly 115. The first handle assembly 105 comprises a cylindrical body having a proximal end and a distal end and a channel 106 extending from a point proximate the proximal end to a point proximate the distal end. A finger ring 110 is positioned at the proximal end of the first handle assembly 105. A cautery pin housing 135 with cautery pin 137 is attached to the distal end of the first handle assembly 105. In one embodiment, the cautery pin housing 135 is rotatable coaxially relative to the first handle assembly 105, increasing user comfort and efficiency. The second handle assembly 115 comprises a center portion with a finger ring 120 on each side. The second handle assembly 115 is coupled to the first handle assembly 105 and slidably movable along the channel 106 of the first handle assembly 105. A collar 125, positioned distal to the second handle assembly 115, is also coupled to the first handle assembly 105 and slidably movable along the channel 106 of the first handle assembly 105.

An elongate tubular sheath 150 having a lumen therein is attached to the distal end of the first handle assembly 105 and extends distally through an opening at the distal end of the cautery tip housing 135. The sheath 150 functions as a drive tube for the snare and a drive wire. In various embodiments, the sheath has an outer diameter ranging from 2.3 to 2.6 mm and an inner diameter ranging from 1.4 to 1.7 mm. In various embodiments, the sheath length is between 2,250 and 2,350 mm. In one embodiment, the sheath length is 2,306 mm. A rigid drive shaft 140 is attached to the distal end of the center portion of the second handle assembly 115 and extends distally through the channel 106 of the first handle assembly 105. In various embodiments, the shaft 140 has an outside diameter between 1.0 and 1.2 mm and an inside diameter between 0.73 and 0.93 mm. In one embodiment, the shaft has an outside diameter of 1.1 mm and an inside diameter of 0.83 mm. The shaft 140 continues through the cautery tip housing 135, wherein it is in electrical communication with the cautery tip 137 and is attached to an elongate flexible drive wire (within sheath 150 described below and not visible in Figure 1). In one embodiment, both the rigid drive shaft 140 and the flexible drive wire are composed of stainless steel 304 (SS304) and nickel-titanium (NiTi). In one embodiment, a length of the sheath 150 just distal to the cautery tip housing 135 includes a shaft strain relief 151. The shaft strain relief 151 overlays the sheath 150 and provides strain relief to the shaft 140 as the shaft 140 is advanced into the sheath 150 to deploy the snare loop. The shaft strain relief 151 ensures smooth movement of the shaft 140 to facilitate proper deployment of the snare loop. In various embodiments, the shaft strain relief 151 has a length between 90 and 100 mm, an outside diameter between 3.1 and 3.3 mm, and an inside diameter between 2.2 and 2.4 mm. In one embodiment, the shaft strain relief 151 has a length of 100 mm, an outside diameter of 3.2 mm, and an inside diameter of 2.3 mm.

The drive wire extends distally within the sheath 150 from the cautery tip housing 135 to a point proximate the distal end of the sheath 150 where it is attached to a snare loop. In one embodiment, the drive wire is comprised of multiple braided wires. In one embodiment, the drive wire includes seven braided wires. In various embodiments, the drive wire has a diameter between 0.65 and 0.85 mm and a length between 2,250 and 2,350 mm. In one embodiment, the drive wire has a diameter of 0.75 mm and a length of 2,294 mm.

In various embodiments, the snare loop also comprises multiple braided wires. In one embodiment, the snare loop comprises seven braided wires. In various embodiments, the snare loop has a diameter between 0.35 and 0.45 mm. In one embodiment, the snare loop has a diameter of 0.40 mm. In one embodiment, the snare loop has a teardrop shape and includes a v-shaped protrusion extending distally from its distal end. In another embodiment, the snare loop has a teardrop shape and does not include a v-shaped protrusion. In various embodiments, the length of the snare loop including the v-shaped protrusion is between 128 and 138 mm from its attachment point to the drive wire to its distal end. In one embodiment, the length of the snare loop including the v-shaped protrusion is 133 mm from its attachment point to the drive wire to its distal end. In various embodiments, the length of the snare loop without the v-shaped protrusion is between 126 and 136 mm from its attachment point to the drive wire to its distal end. In one embodiment, the length of the snare loop without the v-shaped protrusion is 131 mm from its attachment point to the drive wire to its distal end. In various embodiments, a fully deployed snare loop has a maximum operational width between 28 and 32 mm. In one embodiment, a fully deployed snare loop has a maximum operational width of 30 mm.

The wires of the snare loop are attached to the drive wire by a rivet bushing. In the prior art, the bushing is typically crimped onto the wires. In one embodiment of the present specification, the bushing is laser spot/resistance welded to the wires, resulting in no raised edge to drag against the sheath as the snare loop deploys. In one embodiment, the bushing is located approximately 80 mm into the sheath from the distal end when the snare loop is retracted, which is further than what is encountered with prior art snare devices. This ensures smooth drive wire movement to facilitate snare loop deployment and retraction in tortuous anatomy. During manufacture, the drive wire and snare loop are annealed at 490° C for two hours concurrent with loop shape formation. The teardrop shape with notch and annealing process help to maintain the loop configuration during multiple polypectomies in a single procedure, eliminating the 'banana' effect.

In various embodiments, the snare device has a working length between 2,250 and 2,350 mm. In one embodiment, the snare device has a working length of 2,300 mm. In various embodiments, the snare device has a handle deployment throw between 110 and 130 mm and a snare deployment throw of 105 to 109 mm. In one embodiment, the snare device has a handle deployment throw of 120 mm and a snare deployment throw of 107 mm.

FIG. 2 is a side view illustration of the embodiment of the snare device of FIG. 1 with the snare loop withdrawn into the sheath 250, depicting measurement markings 207, 208, 209 printed on the side of the first handle assembly 205. To deploy the snare loop, the collar 225 and second handle assembly 215 are advanced distally along the first handle assembly 205, thereby advancing the shaft and drive wire and deploying the loop past the distal end of the sheath 250. Each measurement marking 207, 208, 209 on the first handle assembly 205 corresponds precisely to the internal diameter or width of the deployed snare loop in millimeters when the distal end of the second handle assembly 215 has been advanced to one of said markings 207, 208, 209. For example, when the distal end of the second handle assembly 215 is moved distally to the '10' marking 207, the deployed snare loop will have a width, at the widest point in the loop, of 10 mm. When the distal end of the second handle assembly 215 is moved further distally to the '20' marking 208, the deployed snare loop will have a width, at the widest point in the loop, of 20 mm and, when the distal end of the second handle assembly 215 is moved even further distally to the '30' marking 209, the deployed snare loop will have a width, at the widest point in the loop, of 30 mm.

The collar 225 is moved just distal to the desired marking 207, 208, 209 before sliding the second handle assembly 215 and functions as a depth stop for the second handle assembly 215 to indicate to the user that he or she has deployed the snare loop the desired amount. Both the collar 225 and the second handle assembly 215 slide along the first handle assembly 205 with some resistance, thereby preventing unintended movement of either component. In one embodiment, each depth marking is denoted by a visible line or, alternatively, an indentation or a raised marking which, when met by the collar 225, generates a clicking sound to indicate to the physician that the precise denoted depth has been reached. Correlating the depth stops precisely with multiple snare loop internal diameters provides the surgeon with a snare device having the functionality of 2 or more prior art devices, thereby alleviating the need of keeping an inventory of different sized snares. In addition, having the ability to deploy a snare loop with multiple, precise widths provides accurate sizing capability of the target tissue to assist the surgeon with immediate follow-up protocol. The collar functioning as a depth stop also provides hybrid throw characteristics of a traditional snare handle and those of a traditional short throw, providing greater versatility and cost efficiency.

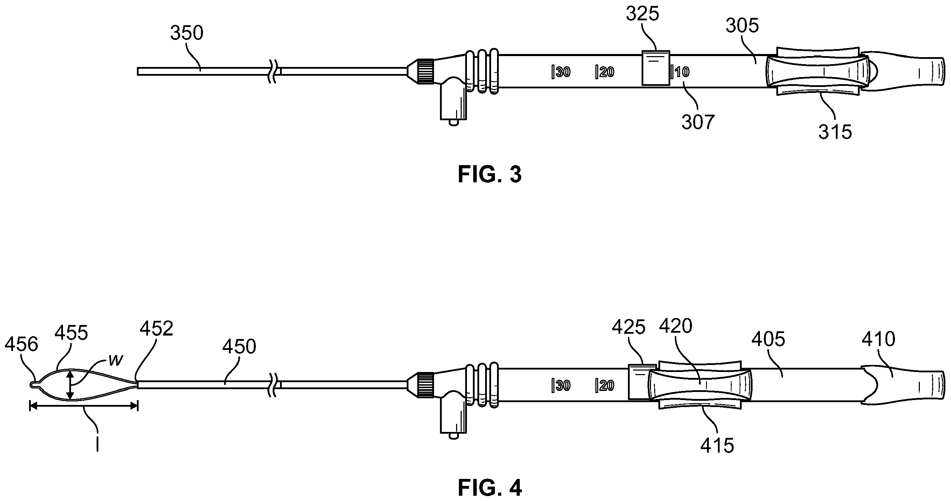

FIG. 3 is a side view illustration of the embodiment of the snare device of FIG. 1 with the snare loop withdrawn into the sheath 350, depicting the collar 325 positioned just distal to the '10' marking 307 on the first handle assembly 305. The second handle assembly 315 is still in its most proximal position and the snare loop is still withdrawn inside the sheath 350. Referring to FIG. 4, the second handle assembly 415 has been slid distally along the first handle assembly 405 until its distal end comes to rest against the proximal end of the collar 425. The collar 425 is in the same position as shown in FIG. 3, though the '10' marking is covered by the second handle assembly 415 in FIG. 4. Advancing the second handle assembly 415 to the position depicted in FIG. 4 deploys the snare loop 455 from the sheath 450 such that the snare loop 455 has an internal diameter or width w of precisely 10 mm, corresponding to the marking on the first handle assembly 405. In addition, when the second handle assembly is at the position depicted in FIG. 4, the snare loop 455 has a length, not including the v-shaped notch 456 discussed below, of 25 mm from the opening 452 at the distal end of the sheath 450 to the distal end of the snare loop 455.

Also visible in FIG. 4 is the v-shaped notch 456 at the distal end of the snare loop. The notch 456 helps the snare loop 455 keep its teardrop shape as it is extended from and retracted into the sheath 450. To deploy the snare loop 455, a user moves the second handle assembly 415 by holding the first handle assembly in place with finger ring 410 and simultaneously using finger rings 420 to slide the second handle assembly 415 distally along the first handle assembly 405. When the snare loop 455 is in the withdrawn position within the sheath 450, the wires of the two ends of the snare loop are positioned parallel to one another. As the snare loop 455 is deployed, the wires twist over each other and then eventually untwist once the snare loop 455 has been deployed to the desired width.

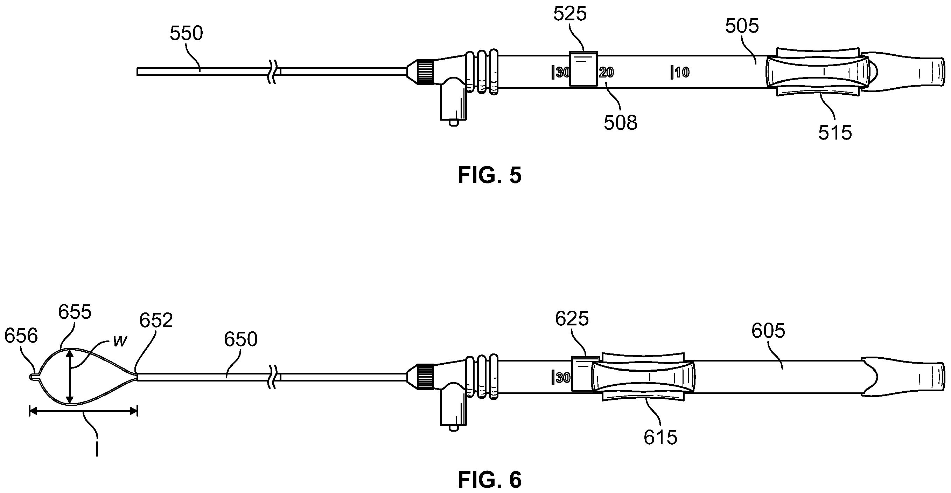

FIG. 5 is a side view illustration of the embodiment of the snare device of FIG. 1 with the snare loop withdrawn into the sheath 550, depicting the collar 525 positioned just distal to the '20' marking 508 on the first handle assembly 505. The second handle assembly 515 is still in its most proximal position and the snare loop is still withdrawn inside the sheath 550. Referring to FIG. 6, the second handle assembly 615 has been slid distally along the first handle assembly 605 until its distal end comes to rest against the proximal end of the collar 625. The collar 625 is in the same position as shown in FIG. 5, though the '20' marking is covered by the second handle assembly 615 in FIG. 6. Advancing the second handle assembly 615 to the position depicted in FIG. 6 deploys the snare loop 655 from the sheath 650 such that the snare loop 655 has an internal diameter or width w of precisely 20 mm, corresponding to the marking on the first handle assembly 605. In addition, when the second handle assembly is at the position depicted in FIG. 6, the snare loop 655 has a length, not including the v-shaped notch 656, of 42 mm from the opening 652 at the distal end of the sheath 650 to the distal end of the snare loop 655.

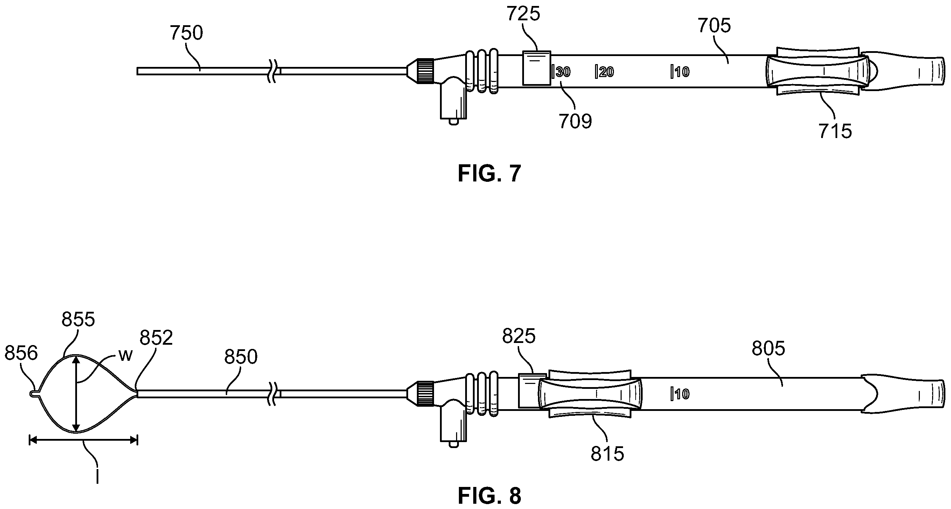

FIG. 7 is a side view illustration of the embodiment of the snare device of FIG. 1 with the snare loop withdrawn into the sheath 750, depicting the collar 725 positioned just distal to the '30' marking 709 on the first handle assembly 705. The second handle assembly 715 is still in its most proximal position and the snare loop is still withdrawn inside the sheath 750. Referring to FIG. 8, the second handle assembly 815 has been slid distally along the first handle assembly 805 until its distal end comes to rest against the proximal end of the collar 825. The collar 825 is in the same position as shown in FIG. 7, though the '30' marking is covered by the second handle assembly 815 in FIG. 8. Advancing the second handle assembly 815 to the position depicted in FIG. 8 deploys the snare loop 855 from the sheath 850 such that the snare loop 855 has an internal diameter or width w of precisely 30 mm, corresponding to the marking on the first handle assembly 805. In addition, when the second handle assembly is at the position depicted in FIG. 8, the snare loop 855 has a length, not including the v-shaped notch 856, of 51 mm from the opening 852 at the distal end of the sheath 850 to the distal end of the snare loop 855.

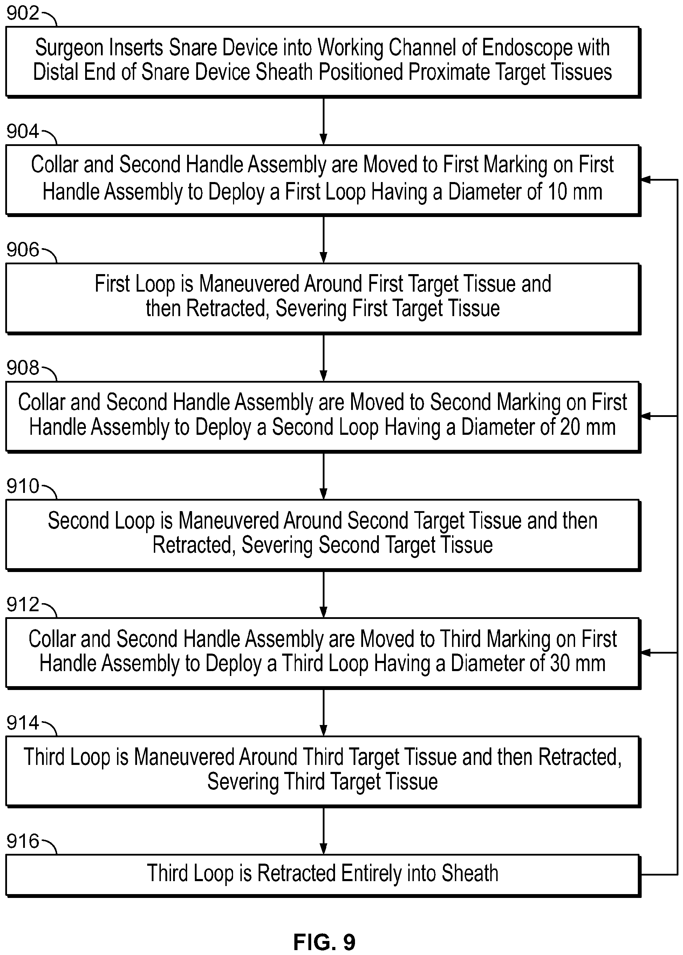

FIG. 9 is a flowchart illustrating one embodiment of the steps included during a multiple polypectomy procedure using the surgical snare device of the present specification. At step 902, a surgeon inserts the snare device into a working channel of an endoscope with the distal end of the sheath positioned proximate target tissues. Then, at step 904, the collar and second handle assembly are moved to the first marking on the first handle assembly to deploy a first loop having a diameter of 10 mm. The first loop is maneuvered around a first target tissue and then retracted, severing the first target tissue at step 906. At step 908, the collar and second handle assembly are moved to the second marking on the first handle assembly to deploy a second loop having a diameter of 20 mm. The second loop is maneuvered around a second target tissue and then retracted, severing the second target tissue at step 910. At step 912, the collar and second handle assembly are moved to the third marking on the first handle assembly to deploy a third loop having a diameter of 30 mm. The third loop is maneuvered around a third target tissue and then retracted, severing the third target tissue at step 914. At step 916, the third loop is retracted entirely into the sheath. At this point, the surgeon can redeploy the snare with a diameter of 10 mm at step 904, with a diameter of 20 mm at step 908, or with a diameter of 30 mm, at step 912 and continue on. The deployment and retraction can be done repeatedly and the loop dimensions will be same each time in correspondence with the marking on the first handle assembly.

The above examples are merely illustrative of the many applications of the system of the present invention. Although only a few embodiments of the present invention have been described herein, it should be understood that the present invention might be embodied in many other specific forms without departing from the scope of the invention. Therefore, the present examples and embodiments are to be considered as illustrative and not restrictive, and the invention may be modified within the scope of the appended claims.

## Claims

1. A surgical snare device for use with an endoscope, said snare device comprising:
a first handle assembly (105) having a cylindrical body with an exterior surface, a proximal end and a distal end, wherein said first handle assembly comprises a first marking (207, 208, 209) having a first value associated therewith and a second marking (207, 208, 209) having a second value associated therewith on said exterior surface of said cylindrical body;
a collar (125) having a cylindrical body with a proximal end and a distal end and, said collar coupled to said first handle assembly around said cylindrical body and slidably movable along said cylindrical body of said first handle assembly such that said collar may be positioned to align with at least one of said first marking and said second marking;
a drive wire having a proximal end and a distal end, wherein said proximal end of said drive wire is attached to an actuator; an elongated tubular sheath (150) attached to the distal end of the first handle assembly; and,
a snare loop (455) attached to said distal end of said drive wire, wherein said snare loop is movable between a first collapsed configuration within the sheath, a second expanded configuration and a third expanded configuration, wherein the second and third expanded configurations deploy past a distal end of said sheath by manipulating said actuator along said first handle assembly such that a portion of said actuator aligns with one of said markings, wherein an internal diameter of said snare loop in the second expanded configuration corresponds to the first value of said first marking and wherein an internal diameter of said snare loop in the third expanded configuration corresponds to the second value of said second marking, **characterized in that**
at least one of said first marking and second marking comprises an indentation or a raised surface which, when physically met by the collar, generates a sound to indicate that a predetermined snare loop internal diameter has been achieved.

2. The snare device of claim 1, wherein the first handle assembly further comprises a third marking having a third value associated therewith and the snare loop is movable to a fourth expanded configuration, wherein the fourth expanded configuration deploys past a distal end of said sheath by manipulating said actuator along said first handle assembly such that a portion of said actuator aligns with the third marking and wherein an internal diameter of said snare loop in the fourth expanded configuration corresponds to the third value of said third marking.

3. The snare device of claim 1, wherein when said actuator portion is positioned at the first marking, the snare loop has a width at its widest point of 10 mm and a length of 25 mm and wherein, when the assembly is positioned at the second marking, the snare loop has a width at its widest point of 20 mm and a length of 42 mm.

4. The snare device of claim 1, further comprising a v-shaped protrusion extending distally at the distal end of the loop and wherein said v-shaped protrusion serves to increase said lengths of the snare loop.

5. The snare device of claim 1 further comprising:
a channel extending from a first point proximate said proximal end of the first handle assembly to a second point proximate said distal end of the first handle assembly;
a second handle assembly (120) having a center body with a proximal end and a distal end, said second handle assembly coupled to said first handle assembly at said channel and slidably movable along said cylindrical body of said first handle assembly between a first position proximate said first point and a second position proximate said second point;
a cautery tip housing (135) having a cautery tip therein, said housing attached to, and rotatable coaxially about, said distal end of said cylindrical body of said first handle assembly; and
a rigid shaft (140) having a proximal end and a distal end and extending through said channel, wherein said proximal end of said shaft is attached to said distal end of said center body of said second handle assembly, further wherein said distal end of shaft is in electrical communication with said cautery tip.

6. The snare device of claim 5, wherein the internal diameter of said expanded snare loop is equal to, in millimeters, the corresponding number aligned with said proximal end of said collar and said distal end of said second handle assembly.

7. The snare device of claim 1, wherein said sheath has an outside diameter ranging from 2.3 to 2.7 mm and an inside diameter ranging from 1.4 to 1.7 mm and/or a length ranging from 2,250 to 2,350 mm.

8. The snare device of claim 5, wherein said shaft has an outside diameter ranging from 1.0 to 1.2 mm and an inside diameter ranging from 0.73 to 0.93 mm.

9. The snare device of claim 1, wherein said drive wire has a diameter ranging from 0.65 to 0.85 mm and/or a length ranging from 2,250 to 2,350 mm.

10. The snare device of claim 1, wherein said snare loop has a teardrop shape and wherein said teardrop shaped snare loop has a length ranging from 126 to 136 mm from its attachment point to the drive wire to the distal end of said snare loop.

11. The snare device of claim 1, wherein said snare loop has a teardrop shape and wherein said teardrop shaped snare loop includes a v-shaped protrusion extending distally from its distal end and wherein said teardrop shaped snare loop with protrusion has a length ranging from 128 to 138 mm from its attachment point to the drive wire to the distal end of said snare loop.

12. The snare device of claim 1, wherein any one or both of said drive wire and said snare loop are composed of a plurality of braided wires.

13. The snare device of claim 12, wherein said plurality of braided wires is equal to seven.

14. The snare device of claim 1, wherein said snare loop is laser spot or resistance welded to said drive wire by a rivet bushing.

15. The snare device of claim 1, further comprising a strain relief sheath attached to and extending distally from said distal end of said cylindrical body of said first handle and overlaying a proximal portion of said elongate tubular sheath.

## Patentansprüche

1. Chirurgische Schlingenvorrichtung zur Verwendung mit einem Endoskop, wobei die Schlingenvorrichtung aufweist:
eine erste Griffanordnung (105) mit einem zylindrischen Körper mit einer Außenfläche, einem proximalen Ende und einem distalen Ende, wobei die erste Griffanordnung eine erste Markierung (207, 208, 209) mit einem ihr zugeordneten ersten Wert und eine zweite Markierung (207, 208, 209) mit einem ihr zugeordneten zweiten Wert auf der Außenfläche des zylindrischen Körpers aufweist;
einen Kranz (125) mit einem zylindrischen Körper mit einem proximalen Ende und einem distalen Ende, wobei der Kranz mit der ersten Griffanordnung um den zylindrischen Körper herum verbunden und entlang des zylindrischen Körpers der ersten Griffanordnung gleitend bewegbar ist, so dass der Kranz positioniert und mit der ersten Markierung und/oder der zweiten Markierung ausgerichtet werden kann;
einen Steuerdraht mit einem proximalen Ende und einem distalen Ende, wobei das proximale Ende des Steuerdrahtes an einem Aktuator befestigt ist;
eine längliche rohrförmige Hülse (150), die am distalen Ende der ersten Griffanordnung befestigt ist; und
eine Schlingenschlaufe (455), die an dem distalen Ende des Steuerdrahtes befestigt ist, wobei die Schlingenschlaufe zwischen einer ersten zusammengelegten Konfiguration innerhalb der Hülse, einer zweiten erweiterten Konfiguration und einer dritten erweiterten Konfiguration beweglich ist, wobei die zweite und die dritte erweiterte Konfiguration durch Manipulieren des Aktuators entlang der ersten Griffanordnung derart, dass ein Teil des Aktuators mit einer der Markierungen ausgerichtet ist, sich jenseits eines distalen Endes der Hülse entfalten , wobei ein Innendurchmesser der Schlingenschlaufe in der zweiten erweiterten Konfiguration dem ersten Wert der ersten Markierung entspricht und ein Innendurchmesser der Schlingenschlaufe in der dritten erweiterten Konfiguration dem zweiten Wert der zweiten Markierung entspricht,
**dadurch gekennzeichnet, dass**
die erste Markierung und/oder die zweite Markierung eine Vertiefung oder eine erhöhte Oberfläche aufweist, die, wenn durch den Kranz physisch getroffen, ein Geräusch erzeugt, das anzeigt, dass ein vorgegebener Innendurchmesser der Schlingenschlaufe erreicht worden ist.

2. Schlingenvorrichtung nach Anspruch 1, wobei die erste Griffanordnung ferner eine dritte Markierung mit einem ihr zugeordneten dritten Wert aufweist und die Schlingenschlaufe zu einer vierten erweiterten Konfiguration bewegbar ist, wobei die vierte erweiterte Konfiguration durch Manipulieren des Aktuators entlang der ersten Griffanordnung derart, dass ein Teil des Aktuators mit der dritten Markierung ausgerichtet ist, sich jenseits eines distalen Endes der Hülse entfaltet, wobei ein Innendurchmesser der Schlingenschlaufe in der vierten erweiterten Konfiguration dem dritten Wert der dritten Markierung entspricht.

3. Schlingenvorrichtung nach Anspruch 1, wobei, wenn der Aktuatorabschnitt an der ersten Markierung positioniert ist, die Schlingenschlaufe an ihrer breitesten Stelle eine Breite von 10 mm und eine Länge von 25 mm hat, und wobei, wenn die Anordnung an der zweiten Markierung angeordnet ist, die Schlingenschlaufe an ihrer breitesten Stelle eine Breite von 20 mm und eine Länge von 42 mm hat.

4. Schlingenvorrichtung nach Anspruch 1, ferner mit einem V-förmigen Vorsprung, der sich distal am distalen Ende der Schleife erstreckt, wobei der V-förmige Vorsprung dazu dient, die Längen der Schlingenschlaufe zu vergrößern.

5. Schlingenvorrichtung nach Anspruch 1, ferner mit:
einem Kanal, der sich von einem ersten Punkt in der Nähe des proximalen Endes der ersten Griffanordnung zu einem zweiten Punkt in der Nähe des distalen Endes der ersten Griffanordnung erstreckt;
einer zweiten Griffanordnung (120) mit einem Mittenkörper mit einem proximalen Ende und einem distalen Ende, wobei die zweite Griffanordnung an dem Kanal mit der ersten Griffanordnung gekoppelt ist und entlang des zylindrischen Körpers der ersten Griffanordnung zwischen einer ersten Position in der Nähe des ersten Punkts und einer zweiten Position in der Nähe des zweiten Punkts gleitend bewegbar ist;
einem Kauterspitzengehäuse (135) mit einer darin angeordneten Kauterspitze, wobei das Gehäuse am distalen Ende des zylindrischen Körpers der ersten Griffanordnung befestigt und koaxial dazu drehbar ist; und
einem starren Schaft (140) mit einem proximalen Ende und einem distalen Ende, der sich durch den Kanal erstreckt, wobei das proximale Ende des Schafts am distalen Ende des Mittenkörpers der zweiten Griffanordnung befestigt ist, wobei ferner das distale Ende des Schafts mit der Kauterspitze elektrisch verbunden ist.

6. Schlingenvorrichtung nach Anspruch 5, wobei der Innendurchmesser der erweiterten Schlingenschlaufe der entsprechenden Zahl in Millimetern gleicht, die mit dem proximalen Ende des Kranzes und dem distalen Ende der zweiten Griffanordnung ausgerichtet ist.

7. Schlingenvorrichtung nach Anspruch 1, wobei die Hülse einen Außendurchmesser im Bereich von 2,3 bis 2,7 mm und einen Innendurchmesser im Bereich von 1,4 bis 1,7 mm und/oder eine Länge im Bereich von 2250 bis 2350 mm hat.

8. Schlingenvorrichtung nach Anspruch 5, wobei der Schaft einen Außendurchmesser im Bereich von 1,0 bis 1,2 mm und einen Innendurchmesser im Bereich von 0,73 bis 0,93 mm aufweist.

9. Schlingenvorrichtung nach Anspruch 1, wobei der Steuerdraht einen Durchmesser im Bereich von 0,65 bis 0,85 mm und/oder eine Länge im Bereich von 2250 bis 2350 mm aufweist.

10. Schlingenvorrichtung nach Anspruch 1, wobei die Schlingenschlaufe eine Tropfenform aufweist, und wobei die tropfenförmige Schlingenschlaufe eine Länge im Bereich von 126 bis 136 mm von ihrem Befestigungspunkt am Steuerdraht bis zum distalen Ende der Schlingenschlaufe aufweist.

11. Schlingenvorrichtung nach Anspruch 1, wobei die Schlingenschlaufe eine Tropfenform aufweist und wobei die tropfenförmige Schlingenschlaufe einen V-förmigen Vorsprung aufweist, der sich distal von ihrem distalen Ende erstreckt, und wobei die tropfenförmige Schlingenschlaufe mit Vorsprung eine Länge im Bereich von 128 bis 138 mm von ihrem Befestigungspunkt am Steuerdraht bis zum distalen Ende der Schlingenschlaufe aufweist.

12. Schlingenvorrichtung nach Anspruch 1, wobei der Steuerdraht und/oder die Schlingenschlaufe aus mehreren geflochtenen Drähten bestehen.

13. Schlingenvorrichtung nach Anspruch 12, wobei die mehreren geflochtenen Drähte sieben Drähte sind.

14. Schlingenvorrichtung nach Anspruch 1, wobei die Schlingenschlaufe durch eine Nietbuchse mit dem Steuerdraht durch Laserpunkt- oder Widerstandsschweißen verbunden ist.

15. Schlingenvorrichtung nach Anspruch 1, ferner mit einer Zugentlastungshülse, die am distalen Ende des zylindrischen Körpers des ersten Griffs befestigt ist und sich distal davon erstreckt und einen proximalen Abschnitt der länglichen rohrförmigen Hülse überlagert.

## Revendications

1. Dispositif à anse chirurgical destiné à être utilisé avec un endoscope, ledit dispositif à anse comprenant :
un premier ensemble formant poignée (105) ayant un corps cylindrique avec une surface extérieure, une extrémité proximale et une extrémité distale, dans lequel ledit premier ensemble formant poignée comprend un premier marquage (207, 208, 209) ayant une première valeur associée à celui-ci et un second marquage (207, 208, 209) ayant une seconde valeur associée à celui-ci sur ladite surface extérieure dudit corps cylindrique ;
un collier (125) ayant un corps cylindrique avec une extrémité proximale et une extrémité distale et,
ledit collier couplé audit premier ensemble formant poignée autour dudit corps cylindrique et pouvant se déplacer par coulissement le long dudit corps cylindrique dudit premier ensemble formant poignée de telle sorte que ledit collier peut être positionné pour s'aligner avec au moins l'un dudit premier marquage et dudit second marquage ;
un fil d'entraînement ayant une extrémité proximale et une extrémité distale, dans lequel ladite extrémité proximale dudit fil d'entraînement est fixée à un organe d'actionnement ;
une gaine tubulaire allongée (150) fixée à l'extrémité distale du premier ensemble formant poignée ; et,
une boucle d'anse (455) fixée à ladite extrémité distale dudit fil d'entraînement, dans lequel ladite boucle d'anse peut se déplacer entre une première configuration affaissée à l'intérieur de la gaine, une seconde configuration expansée et une troisième configuration expansée, dans lequel les seconde et troisième configurations expansées se déploient au-delà d'une extrémité distale de ladite gaine en manipulant ledit organe d'actionnement le long dudit premier ensemble formant poignée de telle sorte qu'une partie dudit organe d'actionnement s'aligne avec l'un desdits marquages, dans lequel un diamètre interne de ladite boucle d'anse dans la seconde configuration expansée correspond à la première valeur dudit premier marquage et dans lequel un diamètre interne de ladite boucle d'anse dans la troisième configuration expansée correspond à la seconde valeur dudit second marquage, **caractérisé en ce qu'**au moins l'un desdits premier et second marquages comprend une empreinte ou une surface relevée qui, lorsqu'elle est physiquement rencontrée par le collier, génère un son pour indiquer qu'un diamètre interne de boucle d'anse prédéterminé a été atteint.

2. Dispositif à anse selon la revendication 1, dans lequel le premier ensemble formant poignée comprend en outre un troisième marquage ayant une troisième valeur associée à celui-ci et la boucle d'anse peut se déplacer dans une quatrième configuration expansée, dans lequel la quatrième configuration expansée se déploie au-delà d'une extrémité distale de ladite gaine en manipulant ledit organe d'actionnement le long dudit premier ensemble formant poignée de sorte qu'une partie dudit organe d'actionnement s'aligne avec le troisième marquage et dans lequel un diamètre interne de ladite boucle d'anse dans la quatrième configuration expansée correspond à la troisième valeur dudit troisième marquage.

3. Dispositif à anse selon la revendication 1, dans lequel lorsque ladite partie d'organe d'actionnement est positionnée au niveau du premier marquage, la boucle d'anse a une largeur à son point le plus large de 10 mm et une longueur de 25 mm et dans lequel, lorsque l'ensemble est positionné au niveau du second marquage, la boucle d'anse a une largeur à son point le plus large de 20 mm et une longueur de 42 mm.

4. Dispositif à anse selon la revendication 1, comprenant en outre une protubérance en forme de v s'étendant de manière distale à l'extrémité distale de la boucle et dans lequel ladite protubérance en forme de v sert à augmenter lesdites longueurs de la boucle d'anse.

5. Dispositif à anse selon la revendication 1 comprenant en outre :
un canal s'étendant d'un premier point à proximité de ladite extrémité proximale du premier ensemble formant poignée à un second point à proximité de ladite extrémité distale du premier ensemble formant poignée ;
un second ensemble formant poignée (120) ayant un corps central avec une extrémité proximale et une extrémité distale, ledit second ensemble formant poignée couplé audit premier ensemble formant poignée au niveau dudit canal et pouvant se déplacer par coulissement le long dudit corps cylindrique dudit premier ensemble formant poignée entre une première position à proximité dudit premier point et une seconde position à proximité dudit second point ;
un boîtier de pointe de cautérisation (135) ayant une pointe de cautérisation à l'intérieur, ledit boîtier fixé à, et pouvant tourner coaxialement autour de, ladite extrémité distale dudit corps cylindrique dudit premier ensemble formant poignée ; et
une tige rigide (140) ayant une extrémité proximale et une extrémité distale et s'étendant dans ledit canal, dans lequel ladite extrémité proximale de ladite tige est fixée à ladite extrémité distale dudit corps central dudit second ensemble formant poignée, en outre dans lequel ladite extrémité distale de la tige est en communication électrique avec ladite pointe de cautérisation.

6. Dispositif à anse selon la revendication 5, dans lequel le diamètre interne de ladite boucle d'anse expansée est égal, en millimètres, au nombre correspondant aligné avec ladite extrémité proximale dudit collier et ladite extrémité distale dudit seconde ensemble formant poignée.

7. Dispositif à anse selon la revendication 1, dans lequel ladite gaine a un diamètre extérieur allant de 2,3 à 2,7 mm et un diamètre intérieur allant de 1,4 à 1,7 mm et/ou une longueur allant de 2 250 à 2 350 mm.

8. Dispositif à anse selon la revendication 5, dans lequel ladite tige a un diamètre extérieur allant de 1,0 à 1,2 mm et un diamètre intérieur allant de 0,73 à 0,93 mm.

9. Dispositif à anse selon la revendication 1, dans lequel ledit fil d'entraînement a un diamètre allant de 0,65 à 0,85 mm et/ou une longueur allant de 2 250 à 2 350 mm.

10. Dispositif à anse selon la revendication 1, dans lequel ladite boucle d'anse a une forme de larme et dans lequel ladite boucle d'anse en forme de larme a une longueur allant de 126 à 136 mm de son point de fixation au fil d'entraînement à l'extrémité distale de ladite boucle d'anse.

11. Dispositif à anse selon la revendication 1, dans lequel ladite boucle d'anse a une forme de larme et dans lequel ladite boucle d'anse en forme de larme comprend une protubérance en forme de v s'étendant de manière distale depuis son extrémité distale et dans lequel ladite boucle d'anse en forme de larme avec protubérance a une longueur allant de 128 à 138 mm de son point de fixation au fil d'entraînement à l'extrémité distale de ladite boucle d'anse.

12. Dispositif à anse selon la revendication 1, dans lequel l'un quelconque ou les deux dudit fil d'entraînement et de ladite boucle d'anse sont composés d'une pluralité de fils tressés.

13. Dispositif à anse selon la revendication 12, dans lequel ladite pluralité de fils tressés est égale à sept.

14. Dispositif à anse selon la revendication 1, dans lequel ladite boucle d'anse est soudée par point laser ou résistance audit fil d'entraînement par une bague de rivet.

15. Dispositif à anse selon la revendication 1, comprenant en outre une gaine de décharge de traction fixée à et s'étendant de manière distale depuis ladite extrémité distale dudit corps cylindrique de ladite première poignée et recouvrant une partie proximale de ladite gaine tubulaire allongée.
